# EUROPEAN PATENT APPLICATION

(11) **EP 1 845 466 A2**
(43) Date of publication of application: **17.10.2007**
(21) Application number: 07251571.1
(22) Date of filing: 12.04.2007
(51) Int. Cl.: G06F 19/00

(54) **Graphical user interface of an external control device for controlling an implantable medical device while minimizing human error**

(30) Priority: 13.04.2006 US 403987
(71) Applicant: Codman Neuro Sciences Sarl, 2400 Le Locle (CH)
(72) Inventor: Ginggen, Alec, 2000 Neuchatel (CH); Utard, Thierry, 2000 Neuchatel (CH); Tardy, Yanik, 2206 les Geneveys-sur-Coffrane (CH); Pipoz, Theirry, 2400 LeLocle (CH); Chossat, Olivier, 25790 Les Gras (FR)
(74) Representative: Tunstall, Christopher Stephen

(57) **Abstract**

A graphical user interface of an external control device for controlling operation of an implantable medical device while minimizing the occurrence of human error. The graphical user interface displays the operating parameter of the implantable medical device simultaneously as an Arabic numeral and a graphical representation. When a parameter value is adjusted, the current and adjusted values are simultaneously displayed as both an Arabic numeral and graphical representation. Other means are described for increasing the probability of detection of an error during entry of an operating parameter value prior to implementation. Execution of the graphical user interface is subject to interruption. When function of the graphical user interface programming is restored operation resumes from that which it left off prior to interruption.

## Description

### BACKGROUND OF THE INVENTION

### Field of the Invention

The present invention is directed to a graphical user interface and, in particular, to a graphical user interface of an external device used to control an implantable medical device, wherein the graphical user interface is designed to reduce the risk of human error while entering and adjusting control parameters as well as during operation.

### Description of Related Art

External devices are widely used to communicate, preferably wirelessly, with an implantable medical device such as a drug infusion pump, sensor or stimulator. During communication the external device is employed to initially set or subsequently adjust parameters for controlling the operation of the implantable medical device. Human error while setting or adjusting parameter values used to control an implantable medical device may result in serious injury, or perhaps even death, to a patient. For example, in the case of a drug infusion pump if an improper dosage level is initially set or subsequently adjusted by the control device then the patient may be over or under medicated both of which may have serious health implications. Heretofore verification procedures utilized to insure the proper setting or adjustment of a parameter value consisted of a confirmation screen that only displayed the entered value once again prior to being implemented. Anyone who has ever proof read a document without even noticing an error therein realizes that this confirmation message alone is by no means an absolute certainty for insuring the detection of an improper parameter value entry.

When initially setting and/or subsequently adjusting parameters of an implantable medical device, even if limited or restricted to only medical personnel, technicians, physicians, or nurses, all too often the user fails to take the time in advance to familiarize themselves with its operating instructions. Furthermore, the operations of different medical devices, even those that perform the same function or task, differ from one manufacturer to another and sometimes from one model to another of the same manufacturer. Therefore, the operator of the implantable medical device may inadvertently confuse the operations of one device with that of another thereby improperly operating the device to the detriment of the health of the patient. Accordingly, it would be desirable to design a system that takes into consideration the fact that the operator or user may not have been properly instructed on the operation of the system or perhaps confuse one system with another.

Considering the substantial life or death risk associated with improper parameter settings by an external device in an implantable medical device system it would be prudent to employ additional precautionary safeguards to minimize the probability of human error. It is therefore desirable to design a graphical user interface for an external control device that provides multiple levels of protection to prevent or substantially reduce the occurrence of human error while operating an implantable medical device as well as during the initial setting and subsequent adjustment of its parameters.

### Summary of the Invention

An object of the present invention is to provide a graphical user interface for an external control device in communication with an implantable medical device which reduces or minimizes the potential for human error during initial setting and/or subsequent adjustment of parameter values as well as ensuring proper operation.

Another object of the present invention is to provide a graphical user interface for an external control device in communication with an implantable medical device that guides the operator or user via step-by-step procedures or active guidance thereby minimizing the probability of human error.

The present invention is directed to a graphical user interface of an external control device for controlling operation of an implantable medical device while minimizing the occurrence of human error. The graphical user interface displays the operating parameter of the implantable medical device simultaneously in multiple visual representations.

One aspect of the invention is directed to a graphical user interface for use with an external control device in communication with an implantable medical device, wherein the graphical user device is programmed to simultaneously display a parameter value of the implantable medical device as a graphical representation and a corresponding Arabic numerical value, preferably side-by-side or one above the other. In the case in which an operating parameter of the implantable medical device is to be adjusted, the graphical user interface programming simultaneously displays a current value and an adjusted value. The current and adjusted parameter values are displayed in real time as two distinct graphical representations and/or corresponding Arabic numerical values.

The graphical user interface for an external control device in communication with an implantable medical device in accordance with the present invention also contemplates programming so that the current and adjusted parameter values are distinguishable from one another, for example, based on color differentiation, font differentiation and/or consistent versus intermittent displaying of values. In addition to the current and adjusted parameter values, the programming of the graphical user interface displays a percentage deviation between the current and adjusted values as well as text classifying the change in parameter value as either an increase or a decrease.

Another aspect of the present invention provides programming of the graphical user interface that minimizes the occurrence of human error when adjusting a parameter value by incrementing/decrementing by a predetermined incremental value the parameter value represented as an Arabic numeral until reaching the desired value. As the Arabic numeral is being incremented/decremented, the adjusted parameter value is simultaneously displayed in real time as a graphical representation. Initially when setting a new parameter value for the first time, the Arabic numeral and associated graphical representation starts at zero and is incremented until reaching the desired value. On the other hand, when subsequently adjusting the parameter value from a current value to an adjusted value, the Arabic numeral and associated graphical representation of the adjusted value are initially displayed as the current value and thereafter incremented/decremented accordingly until reaching the desired adjusted value so as to be readily visually observed as the value is being increased/decreased.

To further reduce the possibility of human error the graphical user interface in accordance with the present invention provides programming for displaying step-by-step guidance for operation of the implantable medical device. The step-by-step guidance may restrict or limit user access to only available operations of the implantable medical device. Furthermore, in another aspect of the invention, the step-by-step guidance displays a message that includes a first portion that requires an active response from at least two possible choices (e.g., affirmative or negative) and a second portion of instructional text associated with the one or more of the at least two choices of the active response prior to an option being selected. The instructional text assists the user in selecting from the response choices prior to entry and may provide text for some or each possible choice.

The last aspect of the present invention is directed to a method for resuming operation of a graphical user interface of an external control device in communication with an implantable medical device. Stored in memory of the implantable medical device is the operation being executed of the graphical user interface of the external control device. An interrupt signal is generated by the external control device. Thereafter, a resume function signal is generated by the external control device. The stored operation of the graphical user interface being executed at the time of the interrupt signal is retrieved from the memory of the implantable medical device and transmitted to the external control device. Execution of the graphical user interface programming for the external control device resumes with the same operation from which it left off at the time of the interrupt signal.

### Brief Description of the Drawing

The foregoing and other features of the present invention will be more readily apparent from the following detailed description and drawings of illustrative embodiments of the invention wherein like reference numbers refer to similar elements throughout the several views and in which:

Figure 1 is an exemplary schematic diagram of an external control device in wireless communication with an implantable medical device, wherein the external control device includes a graphical user interface in accordance with the present invention for setting and adjusting parameters of the implantable medical device while minimizing the probability of human error;

Figures 2a-2i are exemplary screen shots of the external control device display screen generated by the graphical user interface in accordance with the present invention; and

Figure 3 is an exemplary flow diagram that depicts a tracking sequence to ensure that when execution of graphical user interface programming is restored operation resumes from that operation which it left off prior to interrupt or break.

### Detailed Description of the Presently Preferred Embodiments of the Invention

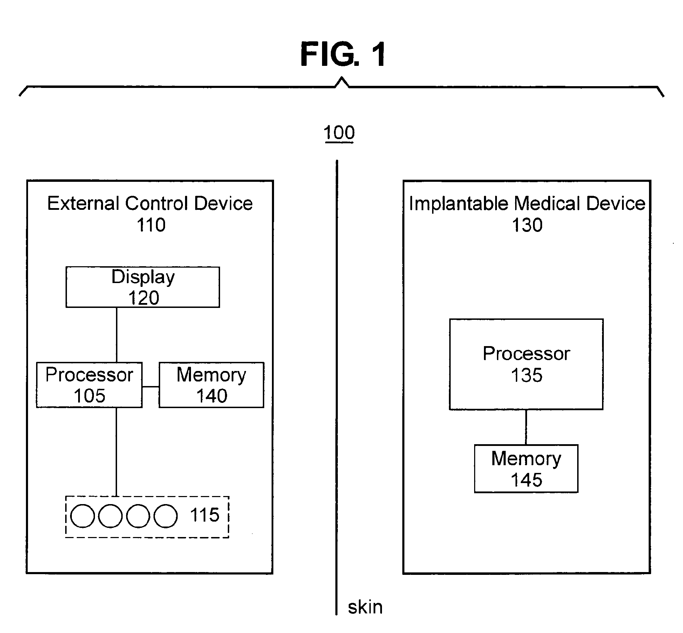

The present invention is directed to a graphical user interface of an external control device that improves safety and reduces human error while setting and thereafter adjusting one or more parameters of a medical device when implanted in the body of a patient. Figure 1 depicts an implantable medical system 100 that includes an implantable medical device 130 in wireless communication with an external control device 110. Preferably, the external control device communicates wirelessly with the implantable medical device thereby eliminating the need for cables or wires therebetween. Despite the clear advantages associated with employing a conventional wireless communication link, the present invention is capable of being used with a wired communication link or any other communication link between the external and implantable devices. It is to be noted, that the present invention is applicable to any type of implantable medical device such as drug infusion pumps, stimulators, sensors or any other device that would benefit by reducing the probability of human error when setting/adjusting operating parameters. A processor or controller 105 associated with the external control device 110 displays operating information on a visual display 120 based on graphical user interface (GUI) programming software stored in memory 140. Any conventional visual display 120 may be used such as a light emitting diode (LED) or liquid crystal diode (LCD) that displays alpha and/or numeric information. Control buttons, knobs or keys 115 are provided on the external control device 110 for setting or adjusting the parameters that control operation of the implantable medical device 130. A mouse, touchpad or any other alternative means may be used to enter or select information viewed on display 120. Depending on the function of implantable medical device 130 the control buttons, knobs or keys 115 may be numeric, alphanumeric and/or directional arrows for orientation of a cursor in the visual display 120. In Figure 1 only four control buttons are shown, however, the number of control buttons, knobs or keys may be altered, as desired.

Substantial interest exists in preventing or substantially reducing errors in the parameter values initially set or subsequently adjusted for controlling the operation of an implantable medical device. To minimize the potential for human error while setting and/or adjusting the operating parameter values of the implantable medical device, the present invention employs at least one, preferably all, distinct safeguard features described in detail below.

One of the most common basis for human error occurs during input or entry of an operating parameter value during its initial setting or subsequent adjustment thereof. Often the user or operator inadvertently pushes the wrong numeric key or button when entering the value. This is especially troublesome with the advent of portable control devices which due to their compact size employ a keypad wherein each key or button has a relatively small surface area. All too often the user or operator depresses or engages more than one key/button or the wrong key/button when entering a particular numeric value. A similar error may occur while manipulating a cursor in making an improper parameter value selection from a menu. Since the entering or selection of an improper parameter value is to be anticipated it is beneficial to contemplate the occurrence of such errors and design the control device 110, and in particular the graphical user interface used to program the display of operating parameter values, in such a way as to increase the probability that any error in the entered parameter value will be readily recognized by the user prior to implementation.

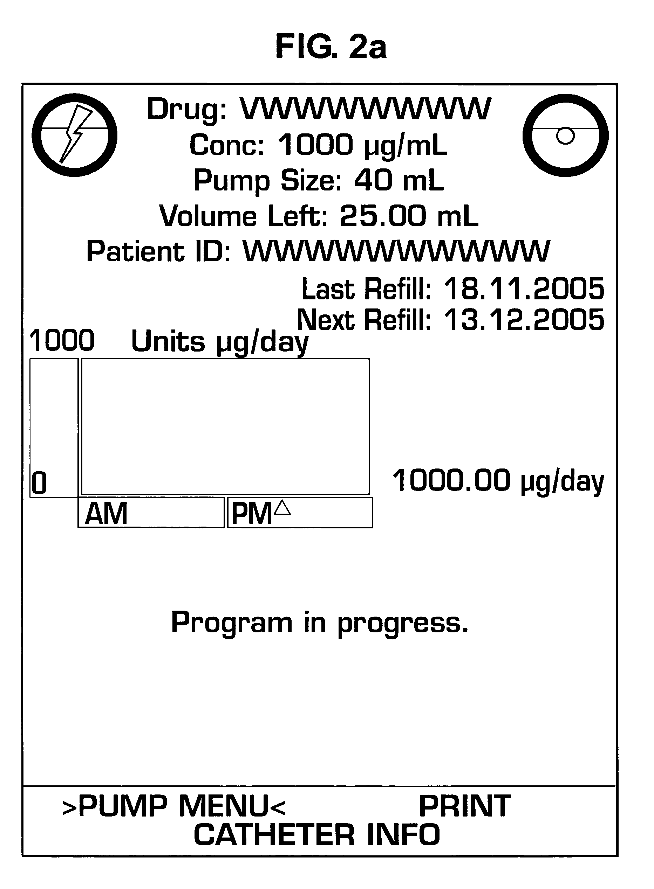

Observation and study has established that an error is apt to be more readily or quickly detected in a visual or graphical representation rather than when viewed only as an Arabic number alone. In keeping with this observation, the present invention contemplates simultaneously displaying the initial setting of and/or subsequent adjustment of a single operating parameter value as both an Arabic number and a graphical representation. Any type of graphical representation may be employed such as a bar graph or pie graph. Figure 2a shows an exemplary display of an initial dosage setting of 1000.00 µg/day for a drug infusion pump. In the screen shot shown in Figure 2a, the initial dosage setting of 1000.00 µg/day is simultaneously represented both as an Arabic number and as a bar graph. Accordingly, by displaying the parameter value in multiple formats the graphical user interface in accordance with the present invention improves the probability of detection of an error by the user or operator when setting and/or adjusting a parameter value of the implantable medical device.

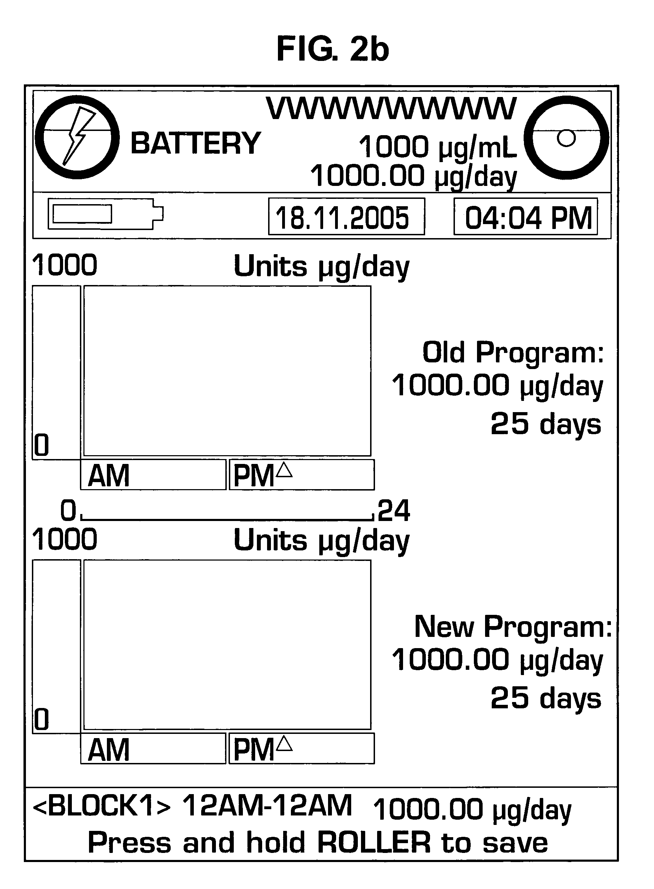

It has also been observed that a visual comparison of at least two graphical representations may increase the probability of error recognition of an operating parameter value by the user. Accordingly, when adjusting a parameter value, the graphical user interface in accordance with the present invention simultaneously displays at least two graphical representations. One graphical representation is of the current operating parameter value, while the other graphical representation depicts the adjusted operating parameter value. Generally, a user visually reads or scans information on a page or screen from top-to-bottom. In the example shown in Figure 2b, the two graphical representations are displayed one above the other wherein the graphical representation of the current operating parameter value ("Old Program") is displayed at the top while the adjusted operating parameter value ("New Program") graphical representation is located at the bottom of the screen. Preferably, the two graphs are displayed in such a manner to further distinguish themselves such as by employing different colors, fonts or some other means for visually differentiating between the current and adjusted operating parameter values. The two graphical representations may be alternatively simultaneously displayed side-by-side. Once again a user generally reads or scans a page or screen from left-to-right. Therefore, the graphical representation of the current operating parameter value is preferably displayed to the left relative to the adjusted operating parameter value graphical representation. These are several desirable configurations, however, the location of the graphical representations may be altered, as desired, so long as both the current and adjusted graphical representations are displayed simultaneously. For instance, the two representations may be depicted in a picture-in-picture (PIP) arrangement. A PIP configuration, however, is not as desirable because the scale or size of the graphical representations are not the same, thereby preventing a direct visual correlation that would readily lead to the detection of an error.

Initially when setting a new parameter value for the first time, the Arabic numeral and associated graphical representation starts at zero and is increased accordingly, in real time, as the user edits the Arabic numeral via the keyboard, cursor and/or mouse. As a result, the user is able to observe the change in graphical representation, in real time, as the Arabic numeral is being entered or incremented.

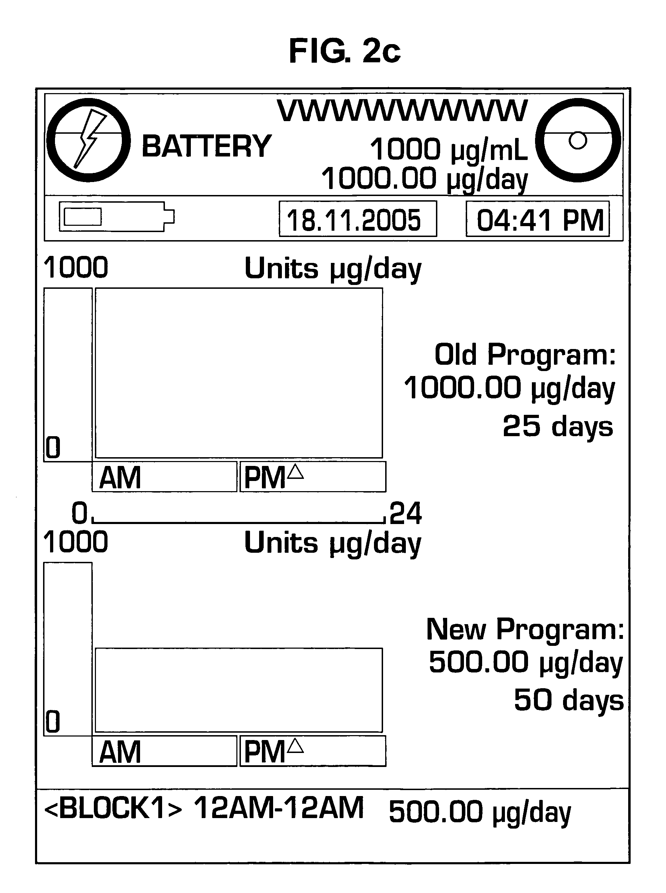

Once an operating parameter has initially been set, thereafter the user may edit or adjust its value. When adjusting an operating parameter value, the graphical user interface is programmed to simultaneously display two graphical representations, one representative of the current operating parameter value ("Old Program") and the other representing the adjusted operating parameter value ("New Program"). Before an adjusted value is entered, the Arabic number and graphical representation for the adjusted operating parameter value is initially displayed as the same value as that of the current operating parameter. Proximate each graphical representation is the corresponding Arabic numeral for the current and adjusted operating parameter values. By way of example, Figure 2b shows an exemplary edit parameter value screen prior to adjustment. In this example, the current dosage value is 1000.00 µg/day. Accordingly, prior to entry of the adjusted dosage value, both the current dosage ("Old Program") and adjusted dosage ("New Program") are equal to 1000.00 µg/day and simultaneously displayed as both an Arabic number and a corresponding graphical representation. Preferably, the adjusted operating parameter value represented both as an Arabic number and graphical representation are depicted in a color, font or some other means for visually differentiating from that of the current operating parameter value so as to be readily observed as the value of interest being edited. For instance, the current operating parameter value may be displayed continuously while the adjusted operation parameter value is displayed intermittently (e.g., toggling on and off). Each digit of the adjusted operating parameter value represented as an Arabic number is preferably changed by incrementing or decrementing its value by one using, for example, the keyboard, pointer and/or mouse. In the example shown in Figure 2c, the adjusted dosage is reduced to 500.00 µg/day. Simultaneously, as the Arabic number of the dosage is adjusted from the current ("Old Program") value of 1000.00 µg/day to the adjusted ("New Program") value of 500.00 µg/day the bar graph associated therewith varies in real time on the screen starting from the current parameter value 1000.00 µg/day and decreasing until reaching a value of 500.00 µg/day. Accordingly, the user is able to readily ascertain visually whether the operating parameter value is increasing/decreasing in value by the bar graph being raised/lowered, respectively. Display of the graphical representation of the operating parameter value in real time allows the user to visually observe the change or adjustment in the value as its associated Arabic number is being entered. Accordingly, a direction of change (e.g., increase/decrease) in the parameter value may be readily recognized as a result of the graphical representation being displayed in real time with adjustment of the parameter value. Accordingly, human error in the direction of change (such as increasing/decreasing the parameter value when it should have been decreased/increased, respectively) will be substantially, perhaps even completely, prevented. Furthermore, a visual comparison of the two graphical representations allows the user to visually observe the relative differential or change in value of the operating parameter.

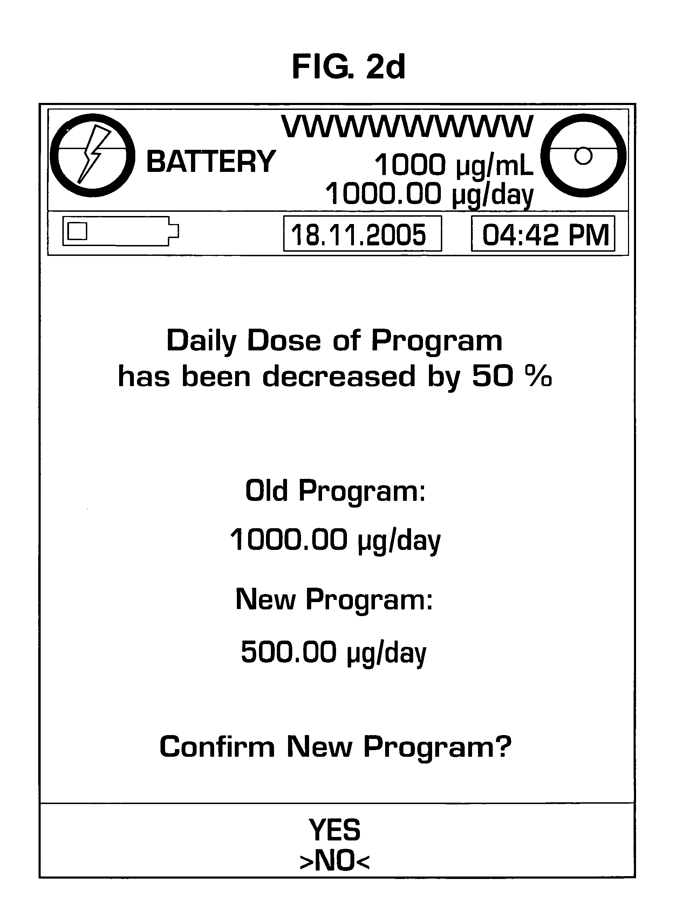

As yet another level of safeguard, a confirmation screen is automatically generated by the graphical user interface in response to entry of the adjusted operating parameter value. An exemplary screen is shown in Figure 2d. The confirmation screen simultaneously displays as an Arabic number both the Old Program (current operating parameter) value of 1000.00 µg/day and the New Program (adjusting operating parameter) value of 500.00 µg/day. In addition, the external control device automatically generates and displays the percentage change in value from the Old Program (current operating parameter) value to the New Program (adjusted operating parameter) value as well as indicating whether the change represents an increase or decrease in value.

The previously described safeguards of the inventive graphical interface system for the external control device have focused on the entry of one or more operating parameter values. Human error is also a significant problem with respect to the overall operation of the control device. Typically, the manufacturer of a control device assumes that the physician, technician, nurse or other personnel has reviewed and understood the instruction manual prior to operating the apparatus or device. Unfortunately, all too often, this is an incorrect assumption. Therefore, the system in accordance with the present invention takes a contradictory approach by assuming that the operator, user, technician, medical personnel, physician or nurse that is using the implantable medical device has less than complete understanding and familiarity with its operation. In keeping with this premise, the graphical user interface provides step-by-step or active guidance screens that prompt or guide the operator each step along the way.

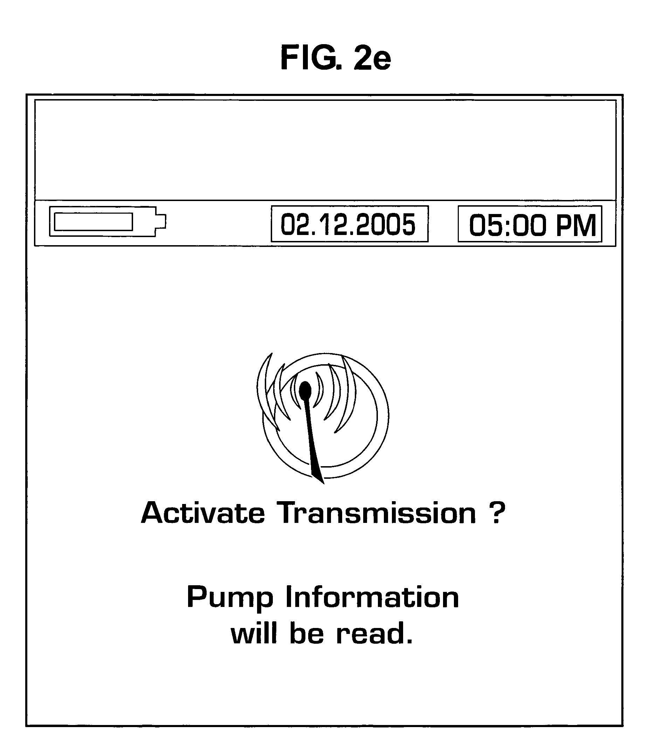

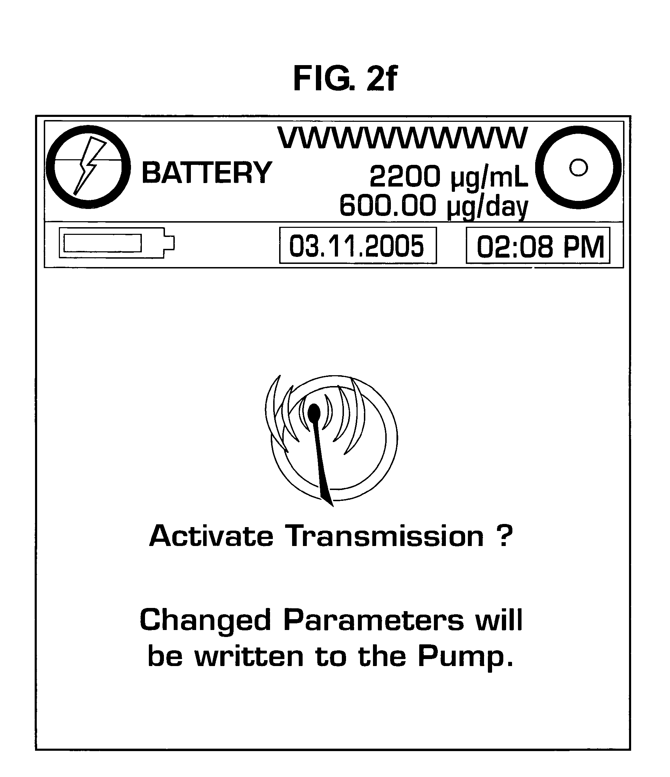

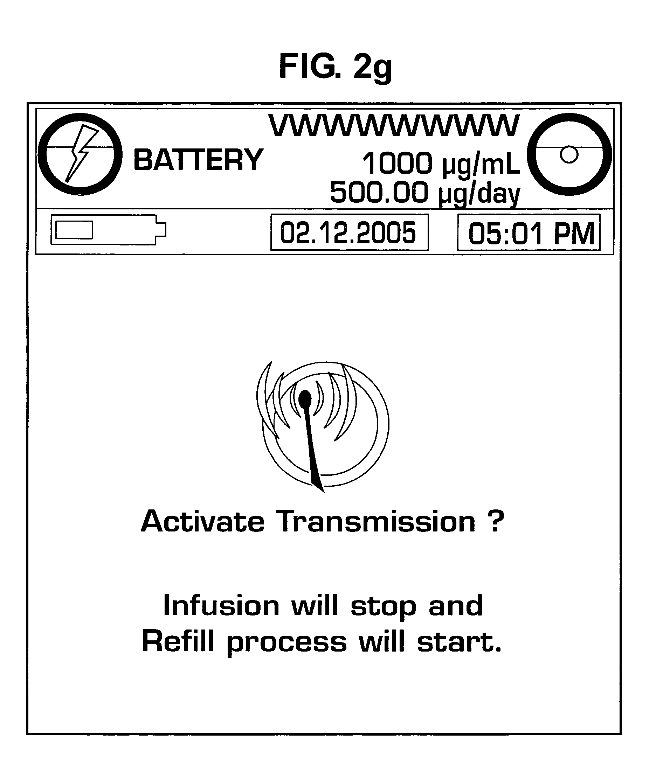

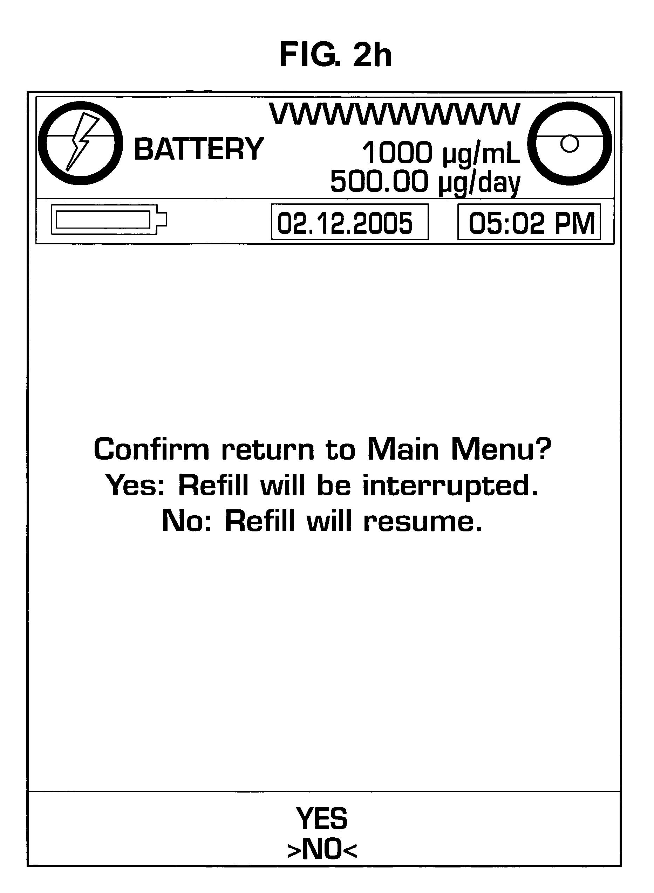

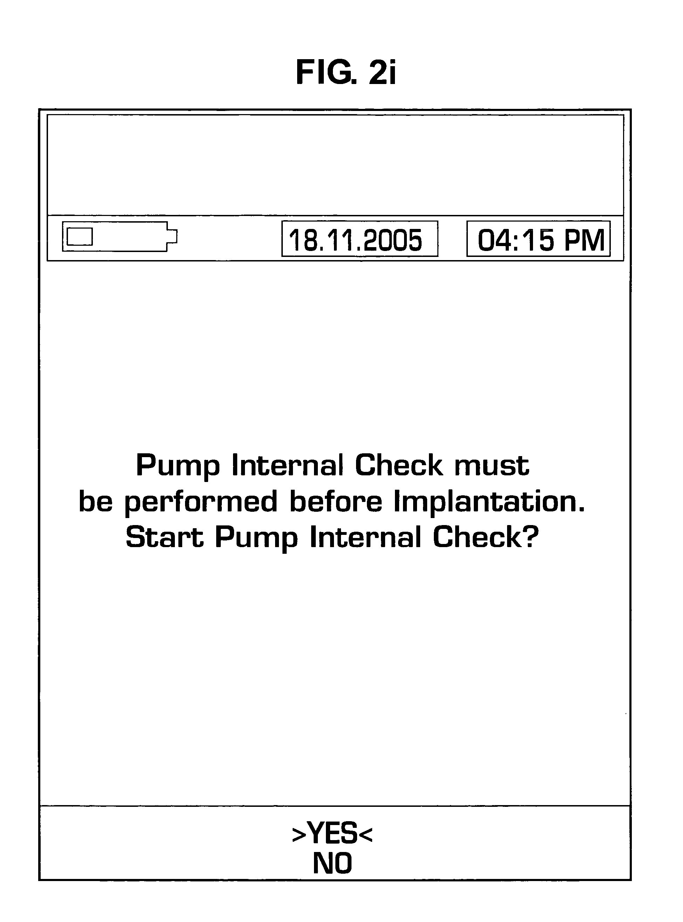

The step-by-step or active guidance can provide the user with instruction information and/or require an affirmative response or action by the user. Some illustrative screen shot examples of the step-by-step or active guidance screens are shown in Figures 2e - 2i. Initially, before the operating parameter values are adjusted the current operating parameters are read from the implantable medical device. Figure 2e shows an exemplary screen that is generated to prompt the user whether to "Activate transmission?" of the reading of data from the implantable medical device. In this example, instructional text is simultaneously displayed to educate the user that in response to receiving an affirmative response (e.g., depressing a button/key of the control device) the "Pump Information will be read." After an operating parameter value has been adjusted a similar screen is generated in Figure 2f which once again prompts the user to confirm by affirmative action whether to "Activate transmission?". However, the instructional text displayed in this screen informs the user that in response to an affirmative response "Changed Parameters will be written to the Pump." Figure 2g shows yet another similar screen generated in response to the user initiating a refill operation in the drug infusion pump during drug infusion. Since infusion operation has to cease prior to initiating refill of the pump, the user is prompted to confirm whether to "Activate Transmission?" by entering some affirmative response. Instructional text displayed on the screen educates the user that in response to the affirmative response "Infusion will stop and Refill Process will start." If during the refill process operation is interrupted, the graphical user interface automatically generates a screen such as that shown in Figure 2h. The user is specifically instructed how the program will proceed depending on which option is selected. If the user confirms returning to the main menu by selecting "yes" then "Refill be interrupted"; otherwise, "Refill will resume." The last example shown in Figure 2i is an instruction screen that educates the user that "Pump Internal Check must be performed before Implantation" and prompts the user whether to "Start Pump Internal Check?". An infinite number of other step-by-step guidance screens can be generated and programmed depending on the particular functions of the implantable medical device. These automatically generated screens guide and prompt the user step-by-step through the operation of the different functions of the implantable medical device in anticipation that the user may not be familiar with or perhaps confused as to how to properly operate the device.

A break or interruption in execution of the graphical user interface programming for the external control device may be necessary for a number of reasons. The term "interrupt signal" is used to denote the signal generated as a result of a break or interruption in execution of the graphical user interface programming. The detection of an error in the programming software for the external control device is one instance in which an interruption and reset is invoked. It is also possible that the user may wish to interrupt execution of the graphical user interface programming in order to recharge the battery source of the external control device or replace/repair the external control device when not operating properly. Another embodiment may contemplate the use of separate interchangeable external control devices, e.g., one used by the patient and the other by a physician, technician or nurse. Any one of these or other circumstances may require the interruption or break in execution of the graphical user interface programming of the external control device. The graphical user interface in accordance with the present invention is designed so that when operation resumes following a break or interruption execution of the programming of the graphical user interface continues from that operation in which it left off at the time of the break or interrupt. By way of example, if the implantable medical device is an implantable drug infusion pump and at the time of interrupt the graphical user interface programming is during refill operation, when function resumes the graphical user interface programming will resume with the refill operation rather than begin at the start of the graphical user interface programming.

In furtherance of this function, the memory device 145 of the implantable medical device 130 is updated or refreshed with the operation of the graphical user interface of the external control device currently being executed. Accordingly, the operation of the graphical user interface for the external control device being executed prior to interruption or break may be retrieved from the memory of the implantable medical device. When function of the external control device resumes the graphical user interface programming continues with the same operation prior to the interrupt or break.

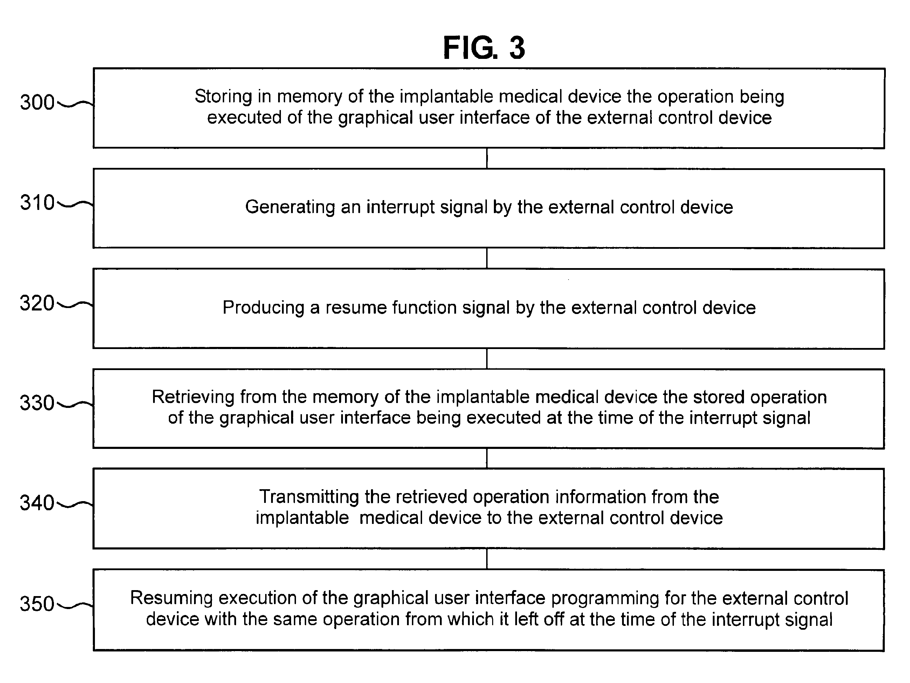

Figure 3 is an exemplary flow chart diagram that depicts the steps performed prior to, during interrupt or break, and thereafter when function of the graphical user interface of the external control device is restored to that operation executed prior to the interrupt or break. Initially, in step 300, the operation of the graphical user interface for the external control device being executed is stored in the memory 145 of the implantable medical device 130. An interrupt signal is generated by the external control device 110 in step 310. As previously noted, the interrupt signal may be generated for any number of reasons including testing/repair of external control device, replacement or recharging of a battery source of the external control device, swapping of external control devices (e.g., patient operated external device versus physician operated external device). Subsequently thereafter, in step 320, the external control device 110 produces a resume function signal. In step 330, the external control device retrieves from the memory of the implantable medical device the stored operation of the graphical user interface being executed at the time of the interrupt or break signal. In step 340, the retrieved operation information is transmitted from the implantable medical device 130 to the external control device 110. Lastly, in step 350 execution of the graphical user interface programming for the external control device resumes with the same operation from which it left off at the time of the interrupt signal. Therefore, in accordance with the present invention, despite an interruption or break in the execution of the graphical user interface programming for the external control device, when function resumes, the graphical user interface continues from that operation prior to the interrupt or break without having to start at the beginning of the programming.

Any one or more of these safeguards may be used with any implantable medical device to reduce the occurrence of human error during operation of an external control device as well as while setting and subsequently adjusting a parameter value by the external control device.

Thus, while there have been shown, described, and pointed out fundamental novel features of the invention as applied to a preferred embodiment thereof, it will be understood that various omissions, substitutions, and changes in the form and details of the devices illustrated, and in their operation, may be made by those skilled in the art without departing from the spirit and scope of the invention. For example, it is expressly intended that all combinations of those elements and/or steps that perform substantially the same function, in substantially the same way, to achieve the same results be within the scope of the invention. Substitutions of elements from one described embodiment to another are also fully intended and contemplated. It is also to be understood that the drawings are not necessarily drawn to scale, but that they are merely conceptual in nature. It is the intention, therefore, to be limited only as indicated by the scope of the claims appended hereto.

Every issued patent, pending patent application, publication, journal article, book or any other reference cited herein is each incorporated by reference in their entirety.

## Claims

1. A graphical user interface for use with an external control device in communication with an implantable medical device, the external control device having a display, the graphical user interface comprising:
programming for displaying simultaneously on the display of the external control device a parameter value of the implantable medical device as a graphical representation and a corresponding Arabic numerical value.

2. A graphical user interface for use with an external control device in communication with an implantable medical device, the external control device having a display, the graphical user interface comprising:
programming for displaying simultaneously on the display of the external control device a current value and an adjusted value for a parameter of the implantable medical device.

3. The graphical user interface in accordance with claim 2, wherein the current and adjusted parameter values are displayed simultaneously as two distinct graphical representations.

4. The graphical user interface in accordance with claim 2, wherein the current and adjusted parameter values are displayed simultaneously as Arabic numerical values.

5. The graphical user interface in accordance with claim 2, wherein the current and adjusted parameter values are displayed simultaneously as both graphical representations and corresponding Arabic numerical values.

6. A graphical user interface for use with an external control device in communication with an implantable medical device, the external control device having a display, the graphical user interface comprising:
programming for displaying on the display of the external control device a current value and an adjusted value for a parameter of the implantable medical device wherein the current value is displayed so as to be visually distinguishable from the adjusted value.

7. The graphical user interface in accordance with claim 6, wherein the current value is displayed as a first color and the adjusted value is displayed as a second color different from the first color.

8. The graphical user interface in accordance with claim 6, wherein the current value is continuously displayed whereas the adjusted value is intermittently displayed.

9. A graphical user interface for use with an external control device in communication with an implantable medical device, the external control device having a display, the graphical user interface comprising:
programming for generating on the display of the external control device a confirmation message that displays a percentage deviation between a current value and an adjusted value for a parameter of the implantable medical device.

10. The graphical user interface in accordance with claim 9, wherein the confirmation message also displays a change in value between the current and adjusted values for the parameter as either an increase or a decrease.

11. A graphical user interface for use with an external control device in communication with an implantable medical device, the external control device having a display, the graphical user interface comprising:
programming for displaying on the display of the external control device step-by-step guidance for operation of the implantable medical device.

12. The graphical user interface in accordance with claim 11, wherein the step-by-step guidance restricts user access to only available operations of the implantable medical device.

13. The graphical user interface in accordance with claim 11, wherein the step-by-step guidance displays a message that includes a first portion that requires an active response of one from at least two choices and a second portion of instructional text based on one or more of the at least two choices of the active response prior to it being received.

14. The graphical user interface in accordance with claim 13, wherein the at least two choices of the active response is an affirmative response and a negative response.

15. The graphical user interface in accordance with claim 13, wherein the instructional text provided is for each choice.

16. A method for entering a parameter value of an implantable medical device using an external control device on which the parameter value is displayed on a display, comprising the steps of:
incrementing/decrementing by a predetermined increment the parameter value represented as an Arabic numeral until reaching the desired value; and
as the Arabic numeral is incremented, simultaneously displaying in real time the graphical representation of the parameter value as it is being incremented.

17. The method in accordance with claim 16, wherein initially when setting a new parameter value for the first time, the Arabic numeral and associated graphical representation starts at zero and is then incremented until reaching the desired value.

18. The method in accordance with claim 16, wherein when adjusting the parameter value from a current value to an adjusted value, the Arabic numeral and associated graphical representation of the adjusted value starts at the current value.

19. A method for resuming operation of a graphical user interface of an external control device in communication with an implantable medical device following an interrupt signal, comprising the steps of:
storing in memory of the implantable medical device the operation being executed of the graphical user interface of the external control device;
generating an interrupt signal by the external control device for interrupting execution of the graphical user interface;
producing a resume function signal by the external control device;
retrieving from the memory of the implantable medical device the stored operation of the graphical user interface being executed at the time of the interrupt signal;
transmitting the retrieved operation information from the implantable medical device to the external control device; and
resuming execution of the graphical user interface programming for the external control device with the same operation from which it left off at the time of the interrupt signal.

20. The method in accordance with claim 19, wherein the interrupt signal is generated in order to (i) interchange external control devices; (ii) replace a power source of the external control device; or (iii) correct for a software error in programming of the external control device.
